Europäisches Patentamt

European Patent Office

Office européen des brevets

(11) Veröffentlichungsnummer: **0 206 265**
**A1**

(12) **EUROPÄISCHE PATENTANMELDUNG**

(21) Anmeldenummer: **86108388.9**

(22) Anmeldetag: **19.06.86**

(51) Int. Cl.4: **B01J 23/78** , **B01J 27/10** , //C07C17/156

(30) Priorität: **22.06.85 DE 3522473**

(43) Veröffentlichungstag der Anmeldung:
**30.12.86 Patentblatt 86/52**

(84) Benannte Vertragsstaaten:
**BE DE NL SE**

(71) Anmelder: **BASF Aktiengesellschaft**
**Carl-Bosch-Strasse 38**
**D-6700 Ludwigshafen(DE)**

(72) Erfinder: **Eichhorn, Hans-Dieter, Dr.**
**Buchnerstrasse 13**
**D-6700 Ludwigshafen(DE)**
Erfinder: **Mross, Wolf Dieter, Dr.**
**Anselm-Feuerbach-Strasse 21**
**D-6710 Frankenthal(DE)**
Erfinder: **Schachner, Helmut, Dr.**
**Zum Grünshof 6**
**D-6909 Walldorf(DE)**
Erfinder: **Schwarzmann, Matthias, Dr.**
**Carl-Bosch-Strasse 54**
**D-6703 Limburgerhof(DE)**
Erfinder: **Cordemans, Luc, Dr.**
**Jozef Mulslaan 13**
**B-2080 Kapellen(BE)**

(54) Geformter Katalysator, Verfahren zu seiner Herstellung und dessen Verwendung bei der Oxichlorierung von Ethylen zu 1,2-Dichlorethan.

(57) Vorgeschlagen wird ein geformter Katalysator mit Kupfer(II)chlorid oder Kupferoxichlorid als aktiver Komponente, dadurch gekennzeichnet, daß er aus

a) 10 bis 95 Gew.% porösem Trägermaterial, auf dessen Oberfläche sich 2 bis 15 Gew.% Kupferionen in Form von Kupfer(II)chlorid und/oder Kupferoxichlorid und 0 bis 5 Gew %. Alkalimetallionen befinden; und

b) 5 bis 90 Gew %. inertem Material. besteht, sowie ein Verfahren zur Herstellung dieses Katalysators.

EP 0 206 265 A1

## Geformter Katalysator, Verfahren zu seiner Herstellung und dessen Verwendung bei der Oxichlorierung von Ethylen zu 1,2-Dichlorethan

Die Erfindung betrifft einen geformten Katalysator mit Kupfer(II)chlorid oder Kupferoxichlorid als aktiver Komponente, ein Verfahren zu seiner Herstellung und dessen Verwendung bei der Oxichlorierung von Ethylen zu 1,2-Dichlorethan.

Die Oxichlorierung von Ethylen zu 1,2-Dichlorethan durch Umsetzung mit Chlorwasserstoff ist ein bekanntes, großtechnisch ausgeübtes Verfahren. Es haben sich insbesondere zwei Verfahren in der Technik eingeführt, bei denen man den Katalysator als Festbett anordnet oder die Reaktion im Fließbett betreibt.

Die Durchführung des Oxichlorierungsverfahrens im Festbett kann entweder ein-oder auch mehrstufig erfolgen. Bei mehrstufiger Durchführung kann Luft bzw. Sauerstoff wie auch Chlorwasserstoff in mehrere Ströme aufgeteilt und jedem Reaktor getrennt zugeführt werden. In weiteren Ausführungsformen sind auch Kreisgasverfahren bekannt, bei denen Teile des aus der Reaktionszone austretenden Gasgemisches, gegebenenfalls nach Abtrennung von 1,2-Dichlorethan und Wasser, mit frischen Einsatzstoffen vermischt, wieder in die Reaktionszone zurückgeführt werden.

Bei der Oxichlorierung von Ethylen finden Katalysatoren Verwendung, die Kupfer(II)chlorid bzw. Kupferoxichlorid auf porösen Trägern wie Aluminiumoxid, Siliciumdioxid, Aluminiumsilikat, Kieselgur oder Bimsstein enthalten. Der Katalysator kann außerdem noch Zusatzstoffe wie Alkalimetallchloride, Metallchloride der seltenen Erden und andere Metallchloride zur Steuerung der Aktivität und der Selektivität enthalten.

Bei der Durchführung eines Oxichlorierungsverfahrens in einem Festbett ist es besonders wichtig, die Temperatur in der Katalysatorschicht zu kontrollieren. Da die Oxichlorierungsreaktion stark exotherm ist, besteht die Gefahr, daß sich lokale, überhitzte Zonen ("hot spots") in der Katalysatorschicht bilden. Diese können zur Zerstörung des Katalysators führen, wodurch der Katalysator zerfällt und der Druckverlust über der Katalysatorschicht ansteigt. Dies führt zur Leistungsverminderung der Oxichlorierungsanlage, so daß der Katalysator unter hohen Kosten ausgewechselt bzw. erneuert werden muß. Außerdem führen zu hohe Temperaturzonen in der Katalysatorschicht zu einem hohen Anteil an unerwünschten Nebenreaktionen, wodurch die Ausbeuten an 1,2-Dichlorethan herabgesetzt werden.

Als Maßnahmen zur Verhinderung oder zumindest zur Verringerung solcher hot spots wurde vorgeschlagen, die Temperatur durch Verdünnen der Einsatzstoffe, durch Variation der Verhältnisse der Einsatzstoffe, durch Verdünnen der Katalysatorteilchen mit Inertmaterial oder durch Variation der Teilchengröße der Katalysatoren und/oder der Inertteilchen zu kontrollieren.

Als Inertmaterial zur Verdünnung der Katalysatoren im Reaktor wurden Siliciumdioxid, Aluminiumoxid, Graphit, Glas oder keramische Teilchen in den verschiedensten Formen wie Tabletten, Kugeln, Ringe oder Stränge vorgeschlagen. Das Verhältnis von Katalysator zu Inertmaterial kann im Reaktor variiert und den Erfordernissen der Reaktion angepaßt werden. Vorzugsweise liegt am Reaktoreingang die höchste Konzentration an Inertmaterial vor, das dann zum Auslaß des Reaktors hin allmählich oder in Stufen abnimmt. Im Reaktor können deshalb auch mehrere Zonen mit unterschiedlichem Verhältnis von Katalysator zu Inertmaterial vorliegen.

Zur Erniedrigung von Temperaturspitzen können auch Katalysatoren mit erniedrigter Aktivität eingesetzt werden, wobei solche Katalysatoren im Reaktor noch zusätzlich mit Inertmaterial abgemischt werden können.

Zur Steuerung bzw. Erniedrigung der Aktivität der Katalysatoren wurden verschiedene Maßnahmen vorgeschlagen. Eine Möglichkeit zur Steuerung der Aktivität besteht in einer Variation der Konzentration an Kupfer(II)chlorid. Katalysatoren mit niedriger Aktivität weisen in der Regel deshalb niedrigere Kupferkonzentrationen als solche mit hoher Aktivität auf. Deshalb nimmt üblicherweise die Kupferkonzentration des Katalysators im Reaktor in Produktstromrichtung zu.

Eine weitere Möglichkeit zur Steuerung der Katalysatoraktivität besteht in der Dotierung mit Alkalimetallsalzen, bevorzugt in Form der Chloride wie Kaliumchlorid, Natriumchlorid, Lithiumchlorid, Rubidiumchlorid, Caesiumchlorid oder Gemische davon. Bis zu 5 Gew.% an Alkalimetall kann im Reaktor enthalten sein, bei höheren Konzentrationen ist die Aktivität dann meist zu gering.

Zur Steuerung der Aktivität der Katalysatoren können auch beide Möglichkeiten, niedrigere Kupferkonzentration und die Dotierung mit Alkali, miteinander kombiniert werden. Der günstigste Fall sollte durch einfache Optimierungsversuche ermittelt werden (s. H.-D. Eichhorn et al., Proc. 8th Int. Congr. Catal. 1984, IV 647-656).

Die Erniedrigung der Katalysatoraktivität für einen Einsatz im Reaktor, wo hohe Temperaturspitzen auftreten, ist aus Selektivitätsgründen nur bis zu einer bestimmten Grenze sinnvoll. Solche Katalysatoren müssen deshalb in der Regel auch noch zusätzlich im Reaktor mit Inertmaterial vermischt

werden. Das Abmischen der Katalysatorpellets mit dem Inertmaterial muß dabei sehr sorgfältig und genau durchgeführt werden, zumal Entmischungsprobleme auftreten, wenn Inertmaterial und Katalysator unterschiedliche Dichten aufweisen. Das Befüllen von technischen Oxichlorierungsreaktoren ist deshalb stets mit einem hohen Zeit-und Kostenaufwand verbunden.

Die relativ hohen thermischen Belastungen, denen Oxichlorierungskatalysatoren des Standes der Technik ausgesetzt sind, sind auch einer der Gründe, warum diese Kontakte mit der Zeit ihre Festigkeit verlieren und damit zum Zerfall neigen, so daß der Druckverlust über der Katalysatorschüttung ansteigt.

Der Erfindung liegt die Aufgabe zugrunde, einen geformten Katalysator mit Kupfer(II)chlorid oder Kupferoxichlorid als aktiver Komponente, sowie ein Verfahren zu seiner Herstellung bereitzustellen, bei dem die vorstehenden Nachteile vermieden werden und ein Katalysator vorliegt, der ohne hohen Kosten-und Zeitaufwand in die Reaktoren eingefüllt und mit verbesserter Standzeit und möglichst geringem Anfall an Nebenprodukten betrieben werden kann.

Diese Aufgabe wird gelöst mit einem Katalysator der genannten Art, der dadurch gekennzeichnet ist, daß er aus (a) 10 bis 95 Gew.% porösem Trägermaterial, auf dessen Oberfläche sich 2 bis 15 Gew.% Kupferionen in Form von Kupfer(II)-chlorid und/oder Kupferoxichlorid und 0 bis 5 Gew.% Alkalimetallionen befinden und (b) 5 bis 90 Gew.% inertem Material besteht.

Gemäß einer bevorzugten Ausführungsform enthält der erfindungsgemäße Katalysator auf dem porösen Trägermaterial 3 bis 10 Gew.% Kupferionen in Form von Kupfer(II)chlorid und/oder Kupferoxichlorid. Es ist bevorzugt, daß der Katalysator 0,2 bis 3 Gew.% eines oder mehrerer Alkalimetallionen, z.B. Natrium-, Kalium-, Lithium-, Rubidium-und/oder Caesiumionen, enthält. Bevorzugt liegen die Alkalimetallionen in Form der entsprechenden Chloride vor. Besonders bevorzugt ist Kaliumchlorid.

Nach einer zweckmäßigen Ausführungsform des erfindungsgemäßen Katalysators macht das inerte Material 10 bis 70 Gew.% des Katalysators aus.

Als inerte Materialien kommen beispielsweise keramische Materialien, Metalloxide oder andere inerte Materialien in Betracht. Beispielsweise kann man nennen: Graphit, Tonerde, Kieselsäure, Titandioxid, Aluminiumsilikat, Siliciumcarbid, Kieselgur, Bimsstein, Zirkondioxid, Glas und keramische Materialien.

Die Erfindung betrifft auch ein Verfahren zur Herstellung des wie zuvor definierten Katalysators, das dadurch gekennzeichnet ist, daß man (a) das poröse Trägermaterial mit der (den) aktiven Komponente(n) tränkt, (b) das imprägnierte Trägermaterial bei Temperaturen im Bereich von etwa 20 bis 300°C trocknet, (c) das getrocknete, imprägnierte Trägermaterial mit dem inerten Material vermischt und (d) das Gemisch zu Formkörpern formt.

Gegenstand der Erfindung ist auch ein geformter Katalysator, der erhältlich ist durch (a) Imprägnieren von porösem Trägermaterial mit 2 bis 15 Gew.%, vorzugsweise 3 bis 10 Gew.%, Kupferionen in Form von Kupfer(II)chlorid und/oder Kupferoxichlorid und 0 bis 5 Gew.%, vorzugsweise 0,2 bis 3 Gew.%, Alkalimetallionen, (b) Trocknen des imprägnierten Trägermaterials, insbesondere bei Temperaturen im Bereich von etwa 20 bis 400°C, (c) Vermischen von 10 bis 95 Gew.% des getrockneten, imprägnierten Trägermaterials mit 5 bis 90 Gew.%, insbesondere 10 bis 70 Gew.% inertem Material und (d) Formen des Gemischs zu Formkörpern.

Die Erfindung betrifft ferner die Verwendung der wie zuvor definierten Katalysatoren zur Oxichlorierung von Ethylen zu 1,2-Dichlorethan in einem Festbett.

Die im vorliegenden Zusammenhang gebrauchte Maßangabe "Gew.%" bezieht sich im Falle der aktiven Komponenten (Kupferionen, Alkalimetallionen) auf das Gesamtgewicht des imprägnierten, porösen Trägermaterials.

Der erfindungsgemäße Katalysator kann gewünschtenfalls auch weitere Zusätze und Zuschlagstoffe enthalten. Beispielsweise seien genannt:

Seltene Erdmetalle, wie Ce, La, Ps, Nd

Elemente der VIII. Nebengruppe des Periodensystems der Elemente, wie Fe, Co, Ni

Erdalkalimetalle, wie Mg, Ca, Sr, Ba.

Derartige Zusätze sind beispielsweise beschrieben in: DE-B-1 417 725, DE-A-2 949 530, PCT/WO81/01284, DE-A-2 613 561, DE-A-2 837 514, DE-B-1 195 726.

Zur Herstellung des erfindungsgemäßen Katalysators mischt man die zuvor genannten Komponenten sowie gewünschtenfalls weitere Zusätze.

Die Komponente (a) (imprägniertes, poröses Trägermaterial) gemäß Anspruch 1 wird hergestellt durch Imprägnieren des porösen Trägermaterials mit Lösungen enthaltend Kupferchlorid und gegebenenfalls Alkaliverbindungen. Das Imprägnieren des Trägers kann nach den üblichen Techniken erfolgen. Zur Tränkung des Trägers können z.B. auch wäßrige oder auch andere, z.B. alkoholische Lösungen von Chloriden und/oder Oxichloriden des Kupfers bzw. der Alkaliverbindungen verwendet werden. Im allgemeinen wird der Träger mit wäßrigen Lösungen der Kupfer-und gegebenenfalls der Alkaliverbindung getränkt, wobei noch andere Zusätze in der Tränklösung, wie z.B. Chlorwasserstoff, enthalten sein können.

Die getränkten Träger können anschließend bei Temperaturen im Bereich von Raumtemperatur bis etwa 300°C an Luft oder auch in Inertgasatmosphäre, z.B. Stickstoff, getrocknet werden, wobei auch eine stufenweise Trocknung von Vorteil sein kann.

Als poröses Trägermaterial können die üblichen Träger wie Kieselgur, Silikagel, Diatomenerde, Bims, Tonerde, Kieselsäure, Silikate und ähnliches verwendet werden. Bevorzugt wird als Trägermaterial Tonerde in Form von $\gamma$-$Al_2O_3$ eingesetzt. Diese Trägerpartikel weisen im allgemeinen eine BET-Oberfläche von 30 bis 350 $m^2$/g auf. Es ist günstig, wenn ein großer Teil der Trägerpartikel eine Größe von weniger als 0,5 mm aufweisen, größere Partikel können vor oder nach dem Tränk-/Trocknungsschritt abgetrennt und eventuell zu kleinen Partikeln gebrochen werden.

Die Herstellung der Mischung aus der Komponente (a) und der Komponente (b) (inertes Material) kann nach den üblichen Verfahren erfolgen. Es kann sich dabei als günstig erweisen, wenn beide Komponenten etwa eine ähnliche Korngrößenverteilung aufweisen. Gewünschtenfalls kann man die Mischung der beiden Komponenten noch in einer Mühle zerkleinern.

Die Verformung des erfindungsgemäßen Katalysators kann nach verschiedenen bekannten Verfahren erfolgen, wie Granulieren, Extrudieren oder Tablettieren.

Bei der Herstellung von rohrförmigen Formkörpern durch Extrudieren kann die Mischung in der Regel mit Wasser und unter Zusatz von Extrudierhilfsmitteln wie Stärke, Methylcellulose, Graphit, Polyethylenglykole, Polyvinylalkohole, Polyvinylpyrrolidone, Polyacrylester, Stearinsäure und deren Metallsalze, Naphthalin, Ammoniak, Ameisensäure, Oxalsäure, Salpetersäure etc. entweder als porositätsverbesserndes Mittel, oder als Gleitmittel oder als Peptisierungsmittel verformt oder zunächst geknetet und anschließend verformt werden; bevorzugt ist dabei die Knetung und die anschließende Verformung, insbesondere das Extrudieren. Die erhaltenen Formlinge werden dann bei Temperaturen im Bereich von Raumtemperatur bis ca. 450°C getrocknet bzw. calciniert, wobei auch eine stufenweise Temperaturbehandlung von Vorteil sein kann.

Bei der Herstellung von rohrförmigen Formkörpern durch Kompressions-Tablettierverfahren können ein oder mehrere Zusätze, wie z.B. Bindemittel und/oder Schmiermittel (Gleitmittel) verwendet werden. Als Beispiele für solche Zusätze seien Graphit, Stearinsäure und deren Metallsalze, Methylcellulose bzw. andere modifizierte Cellulosen, Glycerinmonostearat, Talkum, Polyethylenglykole, Polyvinylpyrrolidone, Polyacrylester u.ä. genannt.

Die Herstellung kugelförmiger Formkörper erfolgt zweckmäßigerweise durch Granulieren unter Zusatz der bekannten Granulierhilfsmittel oder auch durch Tablettieren.

Bevorzugt herzustellende Formkörper sind Tabletten und Ringe, die etwa folgende Abmessungen aufweisen sollen:

Außendurchmesser 4 -12 mm

Innendurchmesser 2 -8 mm

(bei Ringen)

Höhe 3 -12 mm.

Die Durchführung des Oxichlorierungsverfahrens kann ein-oder mehrstufig erfolgen. Bei mehrstufiger Durchführung können auch einzelne Einsatzstoffe, z.B. Luft bzw. Sauerstoff oder Chlorwasserstoff aufgeteilt und den einzelnen Stufen getrennt zugeführt werden. Gegebenenfalls können auch Teile des aus der Reaktionszone austretenden Gemisches, gegebenenfalls nach Abtrennung von 1,2-Dichlorethan und Wasser, mit frischen Einsatzstoffen vermischt, wieder in die Reaktionszone zurückgeführt werden.

Die nachfolgenden Beispiele sollen die Erfindung näher erläutern:

Herstellung der Katalysatoren

Beispiel 1

Die Komponente (a) der erfindungsgemäßen Katalysatoren wurden hergestellt durch Imprägnieren von $\gamma$-Aluminiumoxidpulver, welches eine Korngröße von kleiner als 0,125 mm und eine spezifische Oberfläche von etwa 205 $m^2$/g aufwies, mit einer wäßrigen Lösung von $CuCl_2 \bullet 2 H_2O$ und KCl, wobei die Lösungsmenge etwa der Wasseraufnahmefähigkeit des Trägers entsprach. Die Konzentration an $CuCl_2 \bullet 2 H_2O$ und KCl in der Tränklösung wurde so gewählt, daß die Komponente (a) nach dem Trocknen einen Kupfergehalt von 3,9 Gew.% Kupferionen und einen Kaliumgehalt von 0,45 Gew.% Kaliumionen, bezogen auf die Gesamtmasse der Komponente (a) aufwies. Der imprägnierte Träger wurde anschließend unter Stickstoff zunächst 10 h bei 80°C und danach je 3 h bei 120°C und 210°C getrocknet.

500 g der Komponente (a) wurden danach mit 50 g Graphitpulver intensiv durchmischt und mit einer Tablettiervorrichtung zu Tabletten mit den Maßen 5 mm Höhe und 5 mm Durchmesser verpreßt.

Beispiel 2

500 g der Komponente (a) gemäß Beispiel 1 wurden mit 100 g Graphitpulver intensiv durchmischt und zu 5 ˣ 5 mm Tabletten verpreßt.

Beispiel 3

500 g der Komponente (a) gemäß Beispiel 1 wurden mit 20 g Graphitpulver und 100 g Steatitpulver (Korngröße < 0,2 mm) vermischt und zu 5 ˣ 5 mm Tabletten verpreßt.

Beispiel 4

500 g der Komponente (a) gemäß Beispiel 1 wurden mit 20 g Graphitpulver und 100 g Titandioxidpulver (Korngröße < 0,2 mm) vermischt und zu 5 ˣ 5 mm Tabletten verpreßt.

Beispiel 5

500 g der Komponente (a) gemäß Beispiel 1 wurden mit 20 g Graphitpulver und 100 g Zirkoniumdioxidpulver (Korngröße < 0,2 mm) vermischt und zu 5 ˣ 5 mm Tabletten verpreßt.

Vergleichsbeispiel

Katalysator nach dem Stand der Technik

500 g $\gamma$-$Al_2O_3$-Tabletten mit den Abmessungen 5 mm Höhe und 5 mm Durchmesser wurden mit einer Lösung imprägniert, die $CuCl_2 \cdot 2 H_2O$ und KCl enthielt. Die Konzentration an $CuCl_2 \cdot 2 H_2O$ und KCl in der Tränklösung wurde so gewählt, daß der Katalysator einen Kupfergehalt von 3,9 Gew.% Kupferionen und einen Kaliumgehalt von 0,45 Gew.% Kaliumionen aufwies.

Prüfung der Katalysatoren

Aktivitäts-und Selektivitätstest

Jeweils 90 m³ der erfindungsgemäßen Katalysatoren gemäß Beispielen 1 bis 5 bzw. 90 cm³ einer Mischung aus dem Katalysator des Standes der Technik und 24 Gew.% 5 mm-Kugeln aus keramischem Material wurden in einen Edelstahlreaktor mit 65 mm Innendurchmesser eingefüllt und pro Stunde mit einer Gasmischung beaufschlagt, die 0,986 mole Chlorwasserstoff, 0,493 mole Ethylen und 0,246 mole Sauerstoff in Form von Luft enthielt. Ein Teil des aus der Katalysatorschüttung austretenden Gases wurde mit frischem Einsatzgas vermischt und in die Reaktionszone zurückgeführt. Das Verhältnis von rückgeführtem Reaktionsgas zu frischem Einsatzgas betrug 20:1.

Die Temperatur in der Katalysatorschicht betrug 225°C, der Druck im Reaktor 1 bar. Nach Erreichen eines stationären Betriebszustandes wurden dem aus der Reaktionszone austretenden Gas Proben entnommen und daraus der Umsatz und die Ausbeute an 1,2-Dichlorethan bestimmt. Als Maß für den Umsatz diente der prozentuale Anteil des umgesetzten Chlorwasserstoffs, als Maß für die Ausbeute die Selektivität in Form der prozentualen Anteile vom umgesetzten Ethylen, das zu 1,2-Dichlorethan sowie zu unerwünschten Produkten wie Ethylchlorid, Vinylchlorid, 1,1,2-Trichlorethan sowie zu CO und $CO_2$ umgesetzt worden war. Die Ergebnisse sind in der nachstehenden Tabelle zusammengefaßt.

Standzeiten

Zur Prüfung der Standzeit des Katalysators wurden jeweils 25 cm³ der Katalysatoren gemäß Beispielen 1 bis 5 bzw. 25 cm³ einer Mischung aus dem Katalysator des Standes der Technik mit 24 Gew.% keramischen Kugeln mit einem Durchmesser von 5 mm in einen Edelstahlreaktor mit 10 mm Innendurchmesser eingefüllt. Der Druckverlust der Katalysatorschüttung wurde bei Raumtemperatur bei einer Beaufschlagung von 600 l Stickstoff pro Stunde und einem Druck vor der Schüttung von 1,5 m Wassersäule gemessen.

Danach wurde der Reaktor mit einer Salzschmelze auf 250°C aufgeheizt und der Katalysator pro Stunde mit einem Gas beaufschlagt, welches 16,9 l Chlorwasserstoff, 15,9 l Ethylen und 32,9 l Luft enthielt. Nach 6 Tagen wurde der Reaktor unter Stickstoff abgekühlt und der Druckverlust unter den obigen Bedingungen erneut gemessen. Anschließend wurde der Katalysator ausgebaut und der Anteil an Katalysator bestimmt, der zu Teilchen zerfallen war, die kleiner als 1 mm waren.

Als Maß für die Standzeit des Katalysators wurde der prozentuale Anstieg des Druckverlustes sowie der prozentuale Anteil des Feinanteils im ausgebauten Katalysator angegeben. Die Ergebnisse finden sich in der nachstehenden Tabelle.

Tabelle

| | Bsp.1 | Bsp.2 | Bsp.3 | Bsp.4 | Bsp.5 | Vergleichs-beispiel Katalysator nach dem Stand der Technik |
|---|---|---|---|---|---|---|
| HCl-Umsatz | 68,7 | 67,5 | 67,4 | 67,3 | 68,4 | 67,3 |
| Mol.% des umgesetzten Ethylens zu | | | | | | |
| 1,2-Dichlorethan | 99,23 | 99,35 | 99,09 | 99,15 | 99,14 | 98,52 |
| Vinylchlorid | - | - | - | - | - | 0,03 |
| Ethylchlorid | 0,23 | 0,08 | 0,24 | 0,23 | 0,19 | 0,25 |
| 1,1,2-Trichlorethan | 0,15 | 0,16 | 0,14 | 0,15 | 0,16 | 0,17 |
| $CO + CO_2$ | 0,30 | 0,32 | 0,41 | 0,34 | 0,39 | 0,90 |
| Anstieg des Druckverlustes in % | 28 | 25 | 33 | 24 | 36 | 41 |
| Feinanteil < 1 mm, Gew.% | 7,2 | 6,9 | 7,1 | 6,9 | 7,5 | 7,6 |

Die Ergebnisse der Tabelle zeigen, daß mit den erfindungsgemäßen Katalysatoren erhebliche Vorteile erzielt werden, im Vergleich zu dem Katalysator, der dem Stand der Technik entspricht. Die Ausbeuten an 1,2-Dichlorethan sind bei etwa gleichem HCl-Umsatz wesentlich besser und die Bildung von unerwünschten Nebenprodukten erheblich niedriger. Gleichfalls ist die Standzeit bei Verwendung der neuen Katalysatoren höher, was sich im geringeren Anstieg des Druckverlustes über der Katalysatorschüttung sowie in kleinerem Feinanteil zeigt.

**Ansprüche**

1. Geformter Katalysator mit Kupfer(II)chlorid und/oder Kupferoxichlorid als aktiver Komponente, dadurch gekennzeichnet, daß er aus

a) 10 bis 95 Gew.% porösem Trägermaterial, auf dessen Oberfläche sich 2 bis 15 Gew.% Kupferionen in Form von Kupfer(II)chlorid und/oder Kupferoxichlorid und 0 bis 5 Gew.% Alkalimetallionen befinden; und

b) 5 bis 90 Gew.% inertem Material

besteht.

2. Katalysator nach Anspruch 1, dadurch gekennzeichnet, daß er 3 bis 10 Gew.% Kupferionen in Form von Kupfer(II)chlorid und/oder Kupferoxichlorid enthält.

3. Katalysator nach Anspruch 1 oder 2, dadurch gekennzeichnet, daß er auf dem porösen Trägermaterial 0,2 bis 3 Gew.% eines oder mehrerer Alkalimetallionen enthält.

4. Katalysator nach einem der Ansprüche 1 bis 3, dadurch gekennzeichnet, daß er ein Alkalimetallchlorid enthält.

5. Katalysator nach Anspruch 4, dadurch gekennzeichnet, daß er Kaliumchlorid enthält.

6. Katalysator nach einem der Ansprüche 1 bis 5, dadurch gekennzeichnet, daß er 10 bis 70 Gew.% inertes Material enthält.

7. Verfahren zur Herstellung des Katalysators nach einem der Ansprüche 1 bis 6, dadurch gekennzeichnet, daß man

a) das poröse Trägermaterial mit der (den) aktiven Komponente(n) tränkt,

b) das imprägnierte Trägermaterial bei Temperaturen im Bereich von etwa 20 bis 400°C trocknet,

c) das getrocknete, imprägnierte Trägermaterial mit dem inerten Material vermischt und

d) das Gemisch zu Formkörpern formt.

8. Verfahren nach Anspruch 7, dadurch gekennzeichnet, daß man imprägniertes Trägermaterial und inertes Material mit Wasser anteigt und, gewünschtenfalls unter Zusatz von Extrudierhilfsmitteln, zu Formkörpern extrudiert.

9. Verfahren nach Anspruch 7, dadurch gekennzeichnet, daß man die Mischung aus imprägniertem Trägermaterial und inertem Material, gewünschtenfalls unter Zusatz von Gleit- und/oder Bindemitteln, zu Formkörpern tablettiert.

10. Geformter Katalysator, erhältlich durch

a) Imprägnieren von porösem Trägermaterial mit 2 bis 15 Gew.% Kupferionen in Form von Kupfer(II)chlorid und/oder Kupferoxichlorid und 0 bis 5 Gew.% Alkalimetallionen,

b) Trocknen des imprägnierten Trägermaterials,

c) Vermischen von 10 bis 95 Gew.% des getrockneten, imprägnierten Trägermaterials mit 5 bis 90 Gew.% inertem Material und

d) Formen des Gemisches zu Formkörpern.

11. Verwendung des Katalysators gemäß einem der Ansprüche 1 bis 6 und 10 zur Oxichlorierung von Ethylen zu 1,2-Dichlorethan im Festbett.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 210 593 (SHELL)<br><br>* Ansprüche 11-15; Seite 11, Zeilen 10-19; Seite 22 *<br><br>--- | | B 01 J 23/78<br>B 01 J 27/10 //<br>C 07 C 17/156 |
| A | US-A-3 148 222 (S.E. PENNER)<br><br>* Spalte 3, Zeilen 15,16 *<br><br>--- | | |
| A | FR-A-2 318 135 (STAUFFER CHEMICAL)<br><br>--- | | |
| A | US-A-4 460 699 (R.J. CONVERS)<br><br>----- | | |

RECHERCHIERTE SACHGEBIETE (Int. Cl.4)

B 01 J 23/00
B 01 J 27/00

Der vorliegende Recherchenbericht wurde für alle Patentansprüche erstellt.

| Recherchenort | Abschlußdatum der Recherche | Prüfer |
|---|---|---|
| DEN HAAG | 03-10-1986 | DEVISME F.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82